# EUROPEAN PATENT APPLICATION

(11) **EP 4 556 001 A1**
(43) Date of publication of application: **21.05.2025**
(21) Application number: 24212432.9
(22) Date of filing: 12.11.2024
(51) Int. Cl.: A61K 9/20, A61K 9/24, A61K 31/155, A61K 31/70

(54) **A BILAYER TABLET COMPOSITION COMPRISING DAPAGLIFLOZIN AND METFORMIN**

(30) Priority: 14.11.2023 TR 202314999
(71) Applicant: Sanovel Ilac Sanayi ve Ticaret A.S., Sisli - Istanbul (TR)
(72) Inventor: SUNEL, Fatih, Istanbul (TR); SAHPAZ CERIT, Filiz, Istanbul (TR); GULER, Tolga, Istanbul (TR)
(74) Representative: Genç Ilhan, Oznur

(57) **Abstract**

The invention relates to a bilayer tablet comprising metformin in the form of the free base or in the form of pharmaceutically acceptable salts, crystalline polymorph thereof and dapagliflozin in the form of the free base or in the form of pharmaceutically acceptable salts, crystalline polymorph thereof, and at least one pharmaceutically acceptable excipient, wherein the bilayer tablet has a hardness of more than 253 N.

## Description

The invention relates to a bilayer tablet comprising metformin in the form of the free base or in the form of pharmaceutically acceptable salts, crystalline polymorph thereof and dapagliflozin in the form of the free base or in the form of pharmaceutically acceptable salts, crystalline polymorph thereof, and at least one pharmaceutically acceptable excipient, wherein the bilayer tablet has a hardness of more than 253 N.

### Background of the Invention

Diabetes mellitus, known as diabetes, is a lifelong chronic disease resulting from the pancreas's inability to produce enough insulin or the body's ability to use insulin effectively, and it continues with the reduction of insulin-producing cells. Blood glucose rises with absent or non-functioning insulin hormone. The resulting high blood sugar causes classical symptoms such as polyuria (frequent urination), polydipsia (increased thirst) and polyphagia (increased hunger). Currently, there are three types of diabetes: type 1 diabetes (type 1), type 2 diabetes (type 2) and gestational diabetes. Gestational diabetes is seen in pregnant women who develop high blood sugar levels. Type 1 diabetes is caused by the body's inability to produce insulin, requiring insulin injection. Finally, with type 2 diabetes, the body either resists the effects of insulin or does not produce enough insulin to maintain a normal glucose level. Of these three types of diabetes, type 2 diabetes is the most common type, affecting more than 171 million people worldwide.

Dapagliflozin is a sodium-glucose cotransporter 2 inhibitor (SGLT2). SGLT2 is a carrier responsible for the reabsorption of most of the glucose from the lumen of the renal tubule. SGLT2 is expressed in proximal renal tubules. By inhibiting SGLT2, dapagliflozin reduces the reabsorption of the filtered glucose and lowers the renal threshold for glucose. This improves urinary glucose excretion and blood glucose control. Dapagliflozin, also known as (1S)-1,5-Anhydro-1-C-[4-chloro-3-[(4-ethoxyphenyl)methyl]phenyl]-D-glucitol or (2S,3R,4R,5S,6R)-2-(3-(4-etoxybenzyl)-4-chlorophenyl)-6-hydroxymethyltetrahydro-2H-pyran-3,4,5-triol is represented by the structure of Formula I.

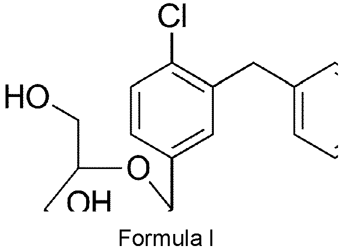

Dapagliflozin was first disclosed in patent US 6515117 (2003, Bristol-Myers Squibb).

Metformin is an antihyperglycemic agent that improves glucose tolerance and lowers both basal and postprandial plasma glucose levels by different mechanisms than other oral antidiabetic agents. Metformin decreases hepatic gluconeogenesis, decreases intestinal absorption of glucose, and improves insulin sensitivity by increasing peripheral glucose uptake and utilization. With metformin therapy, insulin secretion remains unchanged while fasting insulin levels and day-long plasma insulin response may actually decrease. Metformin, also known as N, N-dimethylimidodicarbonimidic diamide or 1,1-Dimethylbiguanide or N, N-dimethyldiguanide or N, N-Dimethylguanylguanidine, is represented by the chemical structure in Formula II.

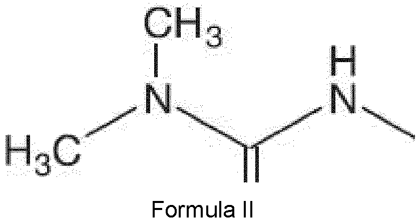

Metformin was first disclosed in patent US 3174901 (1965, Jan Marcel Didier Aron-Samuel).

Bristol-Myers Squibb and AstraZeneca developed another fixed dose combination of Dapagliflozin and Metformin for the treatment of the Type 2 diabetes, wherein Metformin is presented in the extended release formulation and Dapagliflozin is presented in the immediate release formulation. The fixed dose was firstly commercially authorized the U.S. It is currently marketed under the trade name of XIGDUO XR in the strengths of 2.5 mg/1000 mg, 5 mg/500 mg, 5 mg/ 1000 mg, 10 mg/500 mg and 10 mg/ 1000 mg per tablet.

The main advantage of bilayer tablet formulations comprising dapagliflozin or a pharmaceutically acceptable salt thereof with metformin or a pharmaceutically acceptable salt thereof compared to other drug forms is the immediate effect of the drug experienced by the target group of patients, which continues throughout the day. Thus, the target patient group does not need to use drugs repeatedly.

EP2498758B1 discloses bilayer tablets comprising a first layer, wherein the first layer is extended release metformin and a second layer, wherein the second layer is immediate release dapagliflozin molecule.

TR2012/02948 discloses a tablet form comprising a combination of metformin hydrochloride and dapagliflozin and a core tablet coated with a coating solution containing extended release metformin and dapagliflozin.

In the state of the art, there are tablet configurations with layers that provide immediate and extended releases. Immediate release layers balance the amount of glucose in the blood immediately, while the extended-release layers maintain the blood sugar balance over an extended period. But these configurations have undesired properties. Following administration, disintegration and dissolution begin in the immediate and extended-release layers and the active substance diffuses into the environment. However, if the immediate release layer does not diffuse fast enough, the polymers that release and control the release in extended-release layers creates a diffusion barrier surrounding the tablet. This barrier prevents the immediate release layer, which does not release fast enough, from diffusing and the drug from reaching its desired efficiency. As a result, the desired release profile cannot be achieved. The present formulations require additional time for the onset of action. This is undesirable for the patient. The early onset of the treatment process directly affects the patient's quality of life.

In addition, the solubility of the metformin molecule is high whereas the solubility of the dapagliflozin molecule is low. Achieving the intended release profile is quite difficult. Also, dapagliflozin is hygroscopic in nature. It absorbs moisture and forms sticky lumps which are difficult to process and handle, and which may ultimately lead to content uniformity issues in the dosage form. This leads to stability and dissolution profile problems.

In order to eliminate the mentioned problems, it is obvious that an innovation is needed in the art that will facilitate production of daily single-dose formulations with appropriate release profile and stability comprising metformin and dapagliflozin.

There still remains a need in the art to provide a bilayer tablet composition comprising metformin and dapagliflozin. In the present invention, the bilayer tablet composition is created to overcome the above problems and provides additional advantages to the relevant field of art. Other advantages and embodiments of the present invention will be clarified in the following description.

### Background and Description of the Invention

The main object of the present invention is to provide a bilayer tablet composition comprising metformin and dapagliflozin and a method for producing the composition, which have the high stability and excellent extended-release properties with high tablet hardness.

The object of the present invention is to provide a bilayer tablet composition with the desired compressibility and flowability and does not crumble easily, is physically durable.

Metformin is a very poorly compressible active substance and metformin presents in high amounts in a composition. On the other hand, dapagliflozin is used small proportion, also dapagliflozin is hygroscopic in nature. It absorbs moisture and forms sticky lumps which are difficult to process and handle, and which may ultimately lead to content uniformity issues in the dosage form. Because of this situation in the active ingredients, various excipients were used in the composition. However, physical stability is again difficult to achieve. Because too much or too little of the active ingredient created some fluidity and homogeneity problems in the formulation. These problems also lead to problems in the dissolution profile and stability. We have found that forming bilayer tablets at the hardness values specified for stability provides the desired stability and solubility, independent of other conditions.

When the specified hardness was achieved, a hard tablet that did not crumble easily and was resistant to moisture was formed. We have found that this hardness range is important for the desired dissolution profile and stability.

According to an embodiment of the present invention, a bilayer tablet composition comprises;
a. first layer having extended-release comprising metformin in the form of the free base or in the form of pharmaceutically acceptable salts, crystalline polymorph thereof and at least one pharmaceutically acceptable excipient,
b. second layer having immediate release comprising dapagliflozin in the form of the free base or in the form of pharmaceutically acceptable salts, crystalline polymorph thereof, and at least one pharmaceutically acceptable excipient,
wherein the bilayer tablet has a hardness of more than 253 N. the hardness test was done with Erweka machine. The bilayer tablet has excellent tablet hardness, making them difficult to break during molding, transportation, etc., and also have good extended release properties, so they have excellent dissolution control during administration. This situation also positively affects its stability.

According to an embodiment of the present invention, the bilayer tablet has a hardness between 253 N and 450.

Tablets of lower hardness (<253 N) is observed to dissolve very quickly, this is not the result we want for this bilayer tablet. It is observed that tablets with higher hardness (>450 N) could not be broken, so there were problems in dissolution. Thereof, It has been found that the specified values create a suitable condition for the bilayer tablet.

According to an embodiment of the present invention, the bilayer tablet has a hardness between 257 N and 270 N or between 272 N and 285 N or between 286 N and 298 N or between 300 N and 360 N or between 365 N and 430 N.

According to an embodiment of the present invention, the first layer at the bilayer tablet has a hardness between 150 N and 350 N.

According to an embodiment of the present invention, the bilayer tablet further comprises a film coating. After film coating, the bilayer tablet has a hardness of more than 255 N.

According to an embodiment of the present invention, dapagliflozin is present amorphous form, metformin is present hydrochloride form.

According to an embodiment of the present invention, the weight ratio of first layer to second layer is between 2.5 and 4.25. preferably, it is between 3.3 and 3.90.

According to an embodiment of the present invention, the amount of first layer is 70.0% to 82.0% by weight in the total composition. Preferably, it is 73.0% to 80.0% by weight in the total composition.

According to an embodiment of the present invention, the amount of second layer is 10.0% to 25.0% by weight in the total composition. Preferably, it is 18.0% to 23.0% by weight in the total composition.

According to an embodiment of the present invention, the amount of metformin HCl is 30.0% to 45.0% by weight in the total composition.

According to an embodiment of the present invention, the amount of amorphous dapagliflozin is 0.01 % to 5.0% by weight in the total composition.

According to an embodiment of the present invention, at least one pharmaceutically acceptable excipient is selected from fillers, binders, disintegrants, matrix agents, glidants/lubricants or mixtures thereof.

Suitable fillers are selected from the group comprising microcrystalline cellulose (PH 302), anhydrous lactose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

According to an embodiment of the present invention, using filler ensures homogeneous distribution of dapagliflozin, which is used in small amounts in the formulation. The filler is microcrystalline cellulose or anhydrous lactose or mixtures thereof.

According to an embodiment of the present invention, the amount of fillers is 5.0% to 30.0% by weight in the total composition. This ratio provides the desired dissolution profile and content uniformity.

Suitable binders are selected from the group comprising hydroxypropyl methylcellulose, carboxymethylcellulose sodium, polyvinylpyrrolidone (povidone), lactose anhydrous, pregelatinized starch, calcium carbonate, calcium phosphate dibasic, calcium phosphate tribasic, calcium sulphate, hydroxypropylcellulose, methyl cellulose, ethyl cellulose or mixtures thereof.

According to an embodiment of the present invention, the binder is hydroxypropyl methylcellulose (E5 LV) or carboxymethylcellulose sodium.

According to an embodiment of the present invention, the amount of binders is 1.0% to 15.0% by weight in the total composition. Preferably, the amount of binders is 3.0% to 10.0% by weight in the total composition. This provides the desired dissolution profile.

Suitable disintegrants are selected from the group comprising croscarmellose sodium, crospovidone, sodium starch glycollate, sodium alginate, gums, starch and magnesium aluminum silicate or a mixture thereof.

It has been surprisingly found that, according to an embodiment of the present invention, the weight ratio of disintegrant to amorphous dapagliflozin is 1.0 - 4.0, preferably 1.2 - 3.5 in the immediate release layer provides bilayer tablet formulation of dapagliflozin with good dissolution and solubility, thus high bioavailability.

According to an embodiment of the present invention, the disintegrant is croscarmellose sodium.

According to an embodiment of the present invention, the amount of disintegrants is 0.1% to 5.0% by weight in the total composition. This amount of disintegrants provides the desired dissolution profile.

Suitable glidants/lubricants are selected from group comprising colloidal silicon dioxide, sodium stearyl fumarate, magnesium oxide, starch, silica colloidal anhydrous, talc, polyethylene glycol, stearic acid, aluminum silicate, magnesium silicate, colloidal silica or mixtures thereof.

According to an embodiment of the present invention, the glidants/lubricants is colloidal silicon dioxide or sodium stearyl fumarate or mixtures thereof.

According to an embodiment of the present invention, the amount of glidants/lubricants is 0.01% to 6.0% by weight in the total composition.

According to an embodiment of the present invention, the extended release formulation in first layer is prepared using matrix agents.

Suitable matrix agents are selected from the group comprising hydroxypropylmethyl cellulose, ethyl cellulose, polymethylmethacrylate derivatives's, poloxamer, polyvinyl alcohol, xanthan gum, sodium alginate, polymethacrylates, polyacrylamide, hydroxypropyl cellulose, polyvinyl acetate, cellulose acetate phthalate, ethylene vinyl acetate, methyl aminoethyl methacrylate, neutral methacrylic acid esters, polylactide, polylactide co-glycolide, diethyl aminoethyl methacrylate or mixtures thereof.

According to an embodiment of the present invention, the matrix agent is hydroxypropyl methyl cellulose (K100 MCR/ K100M/ CN10T).

According to an embodiment of the present invention, the amount of matrix agents is 10.0% to 25.0% by weight in the total composition.

According to an embodiment of the present invention, the bilayer tablet is coated with at least one film coating agent.

Suitable film coating agents are selected from the group comprising polymethacrylates, hydroxypropyl methylcellulose, lactose monohydrate, talc, hydroxypropyl cellulose, polyvinyl alcohol (PVA), polyethylene glycol (PEG), glycerin, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), iron oxide yellow, iron oxides, all kinds of Opadry^{®}, pigments, dyes, titanium dioxide, coloring agent or mixtures thereof.

According to an embodiment of the present invention, the film coating agents are selected from the group comprising polyvinyl alcohol, talc, titanium dioxide, polyethylene glycol, yellow iron oxide, iron oxide yellow, black iron oxide.

According to an embodiment of the present invention, the amount of film coating agents is 1.0% to 5.0% by weight in the total composition.

In this invention, the combination comprising amorphous dapagliflozin and metformin in the form of the free base or in the form of pharmaceutically acceptable salts, crystalline polymorph thereof is stable. The combination does not show incompatibilities, degradation problems, or extraction problems with certain excipients such as amorphous dapagliflozin, metformin in the form of the free base or in the form of pharmaceutically acceptable salts, crystalline polymorph thereof, microcrystalline cellulose, lactose, croscarmellose sodium, carboxymethylcellulose sodium (7 HF), hydroxypropylmethylcellulose (E5 LV), hydroxypropylmethylcellulose (K100 MCR/ K100M/ CN10T), colloidal silicon dioxide, sodium stearyl fumarate and coating agents.

The present invention provides bilayer tablets comprising an extended release formulation, wherein the first layer comprises metformin in the form of the free base or in the form of pharmaceutically acceptable salts, crystalline polymorph thereof as active ingredient, hydroxypropylmethylcellulose (E5 LV) or carboxymethylcellulose sodium (7 HF) as binder, hydroxypropylmethylcellulose (K100 MCR/ K100M/ CN10T) as a matrix agent, sodium stearyl fumarate as glidant/lubricant, as well as an immediate release formulation of amorphous dapagliflozin wherein the second layer comprises amorphous dapagliflozin as active agent, microcrystalline cellulose and anhydrous lactose as a filler, croscarmellose sodium as disintegrant, colloidal silicon dioxide and sodium stearyl fumarate as glidant/ lubricant.

According to an embodiment of the present invention, the first layer comprises metformin in the form of the free base or in the form of pharmaceutically acceptable salts, crystalline polymorph thereof as active ingredient, hydroxypropylmethylcellulose or carboxymethylcellulose sodium as binder, hydroxypropylmethylcellulose as a matrix agent, sodium stearyl fumarate as glidant/lubricant.

According to an embodiment of the present invention, the second layer comprises amorphous dapagliflozin as active agent, microcrystalline cellulose and anhydrous lactose as a filler, croscarmellose sodium as disintegrant, colloidal silicon dioxide and sodium stearyl fumarate as glidant/ lubricant.

The present invention is described in more detail by the following examples. The example is not to limit the scope of the invention but should be considered in the light of the details described above.

### Example 1:

| **Active ingredient and excipient** | **by weight** |
|---|---|
| **The extended release layer (first layer)** | |
| Metformin HCl | 50.0 - 80.0 |
| Carboxymethylcellulose sodium (7 HF) | 1.0-10.0 |
| Hydroxypropylmethylcellulose (E5 LV) | 0.1 -5.0 |
| Hydroxypropylmethylcellulose (K100 MCR/ K100M/ CN10T) | 10.0 - 25.0 |
| Sodium stearyl fumarate | 0.01 - 3.0 |
| Pure water | q.s. |
| **The extended release layer total** | **75.0 - 85.0** |

| **The immediate release layer (second layer)** | |
|---|---|
| Amorphous dapagliflozin | 0.05 - 5.0 |
| Microcrystalline cellulose | 5.0 - 25.0 |
| Anhydrous lactose | 0.5-6.0 |
| Croscarmellose sodium | 0.1 -5.0 |
| Colloidal silicon dioxide | 0.05 - 3.0 |
| Sodium stearyl fumarate | 0.05 - 3.0 |
| **The immediate release layer total** | **10.0 - 25.0** |

| **Film-coating solution** | |
|---|---|
| Moisture-barrier coating | 1.0-5.0 |
| **The weight of the film tablet (total)** | **100** |

### Example 2:

| **Active ingredient and excipient** | **by weight** |
|---|---|
| **The extended release layer (first layer)** | |
| Metformin HCl | 59.8 |
| Carboxymethylcellulose sodium (7 HF) | 3.0 |
| Hydroxypropylmethylcellulose (E5 LV) | 1.2 |
| Hydroxypropylmethylcellulose (K100 MCR/ K100M/ CN10T) | 16.4 |
| Sodium stearyl fumarate | 0.35 |
| Pure water | q.s. |
| **The extended release layer total** | **80.83** |

| **The immediate release layer (second layer)** | |
|---|---|
| Amorphous dapagliflozin | 0.6 |
| Microcrystalline cellulose | 11.73 |
| Anhydrous lactose | 2.87 |
| Croscarmellose sodium | 0.9 |
| Colloidal silicon dioxide | 0.3 |
| Sodium stearyl fumarate | 0.35 |
| **The immediate release layer total** | **16.77** |

| **Film-coating solution** | |
|---|---|
| Moisture-barrier coating | 2.4 |
| **The weight of the film tablet (total)** | **100** |

### Example 3:

| **Active ingredient and excipient** | **by weight** |
|---|---|
| **The extended release layer (first layer)** | |
| Metformin HCl | 59.8 |
| Carboxymethylcellulose sodium (7 HF) | 3.0 |
| Hydroxypropylmethylcellulose (E5 LV) | 1.2 |
| Hydroxypropylmethylcellulose (K100 MCR/ K100M/ CN10T) | 16.4 |
| Sodium stearyl fumarate | 0.35 |
| Pure water | q.s. |
| **The extended release layer total** | **80.83** |

| **The immediate release layer (second layer)** | |
|---|---|
| Amorphous dapagliflozin | 0.3 |
| Microcrystalline cellulose | 12.0 |
| Anhydrous lactose | 2.87 |
| Croscarmellose sodium | 0.9 |
| Colloidal silicon dioxide | 0.3 |
| Sodium stearyl fumarate | 0.35 |
| **The immediate release layer total** | **16.77** |

| **Film-coating solution** | |
|---|---|
| Moisture-barrier coating | 2.4 |
| **The weight of the film tablet (total)** | **100** |

The above-mentioned pharmaceutical bilayer tablet at example 1 or 2 or 3 is prepared as follows:

**The first layer (extended release layer):**
a) Sieving metformin and grinding,
b) Mixing metformin, carboxymethylcellulose sodium (7 HF), hydroxypropylmethylcellulose (E5 LV) in the granulator and obtained a dry mixture,
c) Spraying water to the dry mixture and then obtained granules,
d) Sieving the granules and then drying at fluid bed dryer,
e) Mixing the granules and hydroxypropylmethylcellulose (K100 MCR/ K100M/ CN10T),
f) Adding sodium stearyl fumarate and then mixing.

**The second layer (immediate release layer):**
g) Mixing amorphous dapagliflozin and colloidal silicon dioxide,
h) Adding anhydrous lactose, croscarmellose sodium and mixing, and then sieving into 630 µ,
i) Adding the half of microcrystalline cellulose and mixing, then adding the remaining part of microcrystalline cellulose and then mixing and then sieving into 630 µ,
j) Adding sodium stearyl fumarate and mixing,

**Tablet compression:** The first layer and second layer are compressed to form a bilayer tablet.

**Film coating:** Film coating with moisture-barrier coating.

With the invention, surprisingly, a bilayer tablet formulation of metformin HCl and amorphous dapagliflozin with the desired release profile is obtained. The immediate release and extended release layers in the bilayer tablet have been used in such proportions, so that a formulation with suitable and desired properties could be obtained which provides up to 24 hours of release. The start of treatment is achieved at the desired level depending on the dissolution rate of the immediate release layer.

## Claims

1. A bilayer tablet composition comprising;
a. first layer having extended-release comprising metformin in the form of the free base or in the form of pharmaceutically acceptable salts, crystalline polymorph thereof and at least one pharmaceutically acceptable excipient,
b. second layer having immediate release comprising dapagliflozin in the form of the free base or in the form of pharmaceutically acceptable salts, crystalline polymorph thereof, and at least one pharmaceutically acceptable excipient,
wherein the bilayer tablet has a hardness of more than 253 N.

2. A bilayer tablet composition according to claim 1, wherein the bilayer tablet has a hardness between 253 N and 450 N.

3. A bilayer tablet composition according to claim 1, wherein the first layer at the bilayer tablet has a hardness between 150 N and 350 N.

4. A bilayer tablet composition according to claim 1, wherein dapagliflozin is present amorphous form, metformin is present hydrochloride form.

5. A bilayer tablet composition according to claim 1, wherein the weight ratio of first layer to second layer is between 2.5 and 4.25.

6. A bilayer tablet composition according to claim 1, wherein at least one pharmaceutically acceptable excipient is selected from fillers, binders, disintegrants, matrix agents, glidants/lubricants or mixtures thereof.

7. A bilayer tablet composition according to claim 6, wherein fillers are selected from the group comprising microcrystalline cellulose (PH 302), anhydrous lactose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

8. A bilayer tablet composition according to claim 6 or 7, wherein the filler is microcrystalline cellulose or anhydrous lactose or mixtures thereof.

9. A bilayer tablet composition according to claim 7 or 8, wherein the amount of fillers is 5.0% to 30.0% by weight in the total composition.

10. A bilayer tablet composition according to claim 6, wherein binders are selected from the group comprising hydroxypropyl methylcellulose, carboxymethylcellulose sodium, polyvinylpyrrolidone (povidone), lactose anhydrous, pregelatinized starch, calcium carbonate, calcium phosphate dibasic, calcium phosphate tribasic, calcium sulphate, hydroxypropylcellulose, methyl cellulose, ethyl cellulose or mixtures thereof.

11. A bilayer tablet composition according to claim 6 or 10, wherein the binder is hydroxypropyl methylcellulose (E5 LV) or carboxymethylcellulose sodium.

12. A bilayer tablet composition according to claim 10 or 11, wherein the amount of binders is 1.0% to 15.0% by weight in the total composition.

13. A bilayer tablet composition according to claim 6, wherein disintegrants are selected from the group comprising croscarmellose sodium, crospovidone, sodium starch glycollate, sodium alginate, gums, starch and magnesium aluminum silicate or a mixture thereof.

14. A bilayer tablet composition according to claim 13, wherein the weight ratio of disintegrant to amorphous dapagliflozin is 1.0 - 4.0.

15. A bilayer tablet composition according to claim 13 or 14, wherein the disintegrant is croscarmellose sodium.
